# EUROPEAN PATENT SPECIFICATION

(11) **EP 1 534 339 B1**
(45) Date of publication and mention of the grant of the patent: **14.10.2009**
(21) Application number: 03787785.9
(22) Date of filing: 13.08.2003
(51) Int. Cl.: A61K 47/10, A61K 47/14, A61K 47/26, A61K 31/445

(54) **Topical anhydrous and ethanol-free pimecrolimus compositions**
Topische wasser-und ethanolfreie Pimecrolimus enthaltende Zusammensetzungen
Compositions de pimécrolimus anhydres topiques et dépourvues d'éthanol

(30) Priority: 14.08.2002 GB 0218996
(43) Date of publication of application: 01.06.2005
(73) Proprietor: Novartis AG, 4056 Basel (CH); Novartis Pharma GmbH, 1230 Wien (AT)
(72) Inventor: LEDERGERBER, Dorothea, 79594 Inzlingen (DE); SONNTAG, Jean-Claude, F-68260 Kingersheim (FR); KRIWET, Katrin, 79639 Grenzach-Wyhlen (DE); SEKKAT, Nabila, CH-4056 Basel (CH)
(74) Representative: Grubb, Philip William
(86) International application number: PCT/EP2003/008999
(87) International publication number: WO 2004/016289

(56) References cited:
- EP-A- 1 074 255
- EP-A- 1 147 766
- WO-A-96/13249

## Description

The invention relates to topical pharmaceutical compositions comprising pimecrolimus for the treatment of skin disorders, in the form of a single-phase liquid or semi-solid composition substantially free of ethanol and water.

Ascomycins such as ascomycin itself or derivatives thereof arc examples of compounds of the FK 506 class. FK506 (tacrolimus) is a known macrolide antibiotic that is produced by Streptomyces tsukubaensis No. 9993. It is also a potent immunosuppressant. The structure of FK506 is given in The Merck Index 12th Edition (1996) on p. 546 as entry 9200. Methods of preparing FK506 are described in e.g. EP 184162.

A large number of derivatives, antagonists, agonists and analogues of FK506, which retain the basic structure and at least one of the biological properties (for example the immunological properties) of FK506, are now known. These compounds are described in a large number of publications, for example EP 184162, EP 315978, EP 323042, EP 423714, EP 427680, EP 465426, EP 474126, WO 91/13889, WO 91/19495, EP 484936, EP 532088, EP 532089, EP 569337, EP 626385 and WO 93/5059. Ascomycin and derivatives thereof, including FK506, are referred to hereinafter as "ascomycins".

Ascomycins are known to be indicated for use in e.g. the topical treatment of inflammatory and hyperproliferative skin diseases and of cutaneous manifestations of immunologically-mediated illnesses. The condition of the skin to be treated may vary e.g. with kind and grade of disease; e.g. the skin maybe dry or fatty skin. Furthermore, the skin to be treated may be haired.

Abstract WPI, JP913379 discloses a composition substantially free of ethanol and water for topical administration comprising pimecrolimus (33-epichloro-33-desoxy-ascomycin), i) propylene glycol, ii) oleyl alcohol, and a thickening agent such as polyethylene glycol or a carboxyvinyl polymer.

EP-A 1 074 255 discloses a pharmaceutical composition comprising a macrolide compound such as, for example, 33-epi-chloro-33-desoxyascomycin, or its pharmaceutically acceptable salt, i) propylene glycol, ii) oleyl glycol and iii) polyethylene glycol (4.5 weight%)..

WO 96/13249 and EP-A 1 147 766 disclose dermatologic ascomycin compositions in form of emulsions containing oleyl alcohol, miglyol, hexylene glycol and water.

There is a need for a pharmaceutical composition that may be applied to the skin substantially independently of the condition of the skin.

It has now been found that an ascomycin can be formulated into a pharmaceutical composition in the form of a single-phase liquid or semi-solid topical formulation substantially free of ethanol and water. These pharmaceutical compositions are effective independently of the condition of the skin, nail or mucosa, are well tolerated, stable and have particularly interesting penetration properties.

They surprisingly retain and improve on the beneficial penetration properties of more complex or inhomogenous formulations such as water- or hydrocarbon-based emulsions or suspensions, while being particularly convenient in terms of ease of administration and patient compliance.

Thus it has been found that a large part of the bulk of the compositions, at least 40 % w/w, can consist of simple solvents, resulting in easily-applicable, clear liquid solutions or thickened, semi-solid solutions.

In particular, the invention discloses a **single-phase topical liquid** or semi-solid pharmaceutical composition substantially **free of ethanol and water** which comprises an ascomycin in a carrier vehicle comprising **a 3-component solvent mixture** amounting to at least 40 **% w/w of the total weight** of the composition and consisting of:
i) a **C₃₋₈ alkanol** and/or **C₁₋₈ alkanediol;**
ii) a **fatty alcohol;** and
iii) a **further solvent** selected from:
   a) an **alkane carboxylic acid alkyl** ester and/or **alkane dicarboxylic acid alkyl** ester and/or
   b) a **hydrophilic co-component** and/or
   c) a **triglyceride;**
and optionally further conventional excipients.

The present invention relates to a single-phase topical liquid or semi-solid pharmaceutical composition substantially free of ethanol and water which comprises pimecrolimus in a carrier vehicle comprising a 3-component solvent mixture amounting to at least 40% w/w of the total weight of the composition and consisting of:
i) isopropanol and/or hexylene glycol;
ii) oleyl alcohol; and
iii):
   a) diisopropyl adipate and/or isopropyl myristate and/or
   b) dimethyl isosorbide and/or
   c) medium-chain triglycerides;
and optionally further conventional excipients, hereinafter briefly named "**the compositions of the invention".**

The compositions of the invention have the advantage that they consist of few components, are straightforward to prepare and are well-tolerated on human skin.

The compositions of the invention may be e.g. in the form of a topical solution or a spray solution, a gel, a foam or an ointment, preferably they are in the form of a gel, a foam or an ointment.

Semi-solid or foamy compositions are preferred.

"Single-phase" means herein that the compositions of the invention are other than multiphasic, e.g. other than two-phase systems such as creams or emulsions; they may be in any single-phase form, e.g. as a solution or spray solution, a gel, an ointment or e.g. the liquid component of a foam, whereby the active ingredient normally is in dissolved form.

"Liquid or semi-liquid" means that the compositions of the invention are essentially solutions in liquid or solidified liquid form.

"Substantially free of ethanol and water" means that neither ethanol nor water is added as an intentional constituent part of the compositions of the invention. However, e.g. a small amount of humidity, e.g. up to about 1 % water w/w, may nevertheless be present, e.g. as an intrinsic impurity in some of the excipients used, or as part of the active ingredient when this is e.g. a hydrate, e.g. when crystal form A (see WO 99/01458) of compound A hereinafter is used.

A particularly beneficial aspect of the compositions of the invention is that while the components of the above 3-component solvent mixture are solubilizing agents, they additionally may possess penetration enhancing properties, thus contributing to keeping the formulations both simple and effective. Further, it has been found that the solutions may be thickened without any apparent loss in efficacy.

Ascomycins disclosed herein include e.g.:
- FK506 **(tacrolimus);**
- 32-[4-(3,5-dimethoxyphenyl)imidazol-1-ylmethoxy]ascomycin **(L-733725)** (in Example 1 and as compound of formula I in WO 97/8182);
- 32-O-(1-hydroxyethylindol-5-yl)ascomycin **(L-732531)** (Transplantation 65 [1998] 10-18, 18-26, Figure 1 on page 11);
- (32-desoxy-32-epi-N1-tetrazolyl)ascomycin **(ABT-281)** (J. Invest. Dermatol. 12 [1999] 729-738, Figure 1 on page 730);
- 33-epichloro-33-desoxy-ascomycin **(pimecrolimus),** i.e. {[1E-(1R,3R,4S)]1R,9S,12S,13R, 14S,17R,18E,21S,23S,24R,25S,27R}-12-[2-(4-chloro-3-methoxycyclohexyl)-1-methylvinyl]-17-ethyl-1,14-dihydroxy-23,25-dimethoxy-13,19,21,27-tetramethyl-11,28,dioxa-4-azatricyclo [22.3.1.0(4,9)]octacos-18-ene-2,3,10,16-tetraone, of formula I 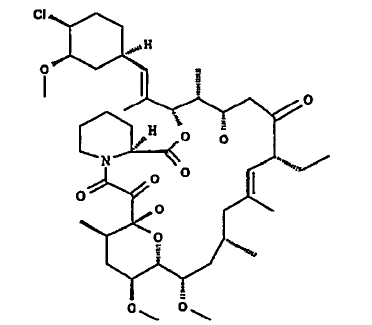 disclosed as Example 66a in e.g. EP 427680 (hereinafter referred to as Compound A);
- {[1E-(1R,3R,4R)]1R,4S,SR,6S,9R,10E,13S,15S,16R,17S,19S,20S}-9-ethyl-6,16,20-trihydroxy-4-[2-(4-hydroxy-3-methoxycyclohexyl)-1-methylvinyl]-15,17-dimethoxy 5,11,13,19-tetramethyl-3-oxa-22-azatricyclo[18.6.1.0(1,22)]heptacos-10-ene-2,8,21,27-tetraone disclosed as Examples 6d and 71 in e.g. EP 569337 (hereinafter referred to as **Compound B**); and
- {1R,5Z,9S,12S-[1E-(1R,3R,4R)],13R,14S,17R,18E,21S,23S,24R,25S,27R}17-ethyl-1,14-dihydroxy-12-[2-(4-hydroxy-3-methoxycyclohexyl)-1-methylvinyl]-23,25-dimethoxy 13,19,21,27-tetramethyl-11,28-dioxa-4-azatricyclo[22.3.1.0(4,9)]octacosa-5,18-diene-2,3,10,16-tetraone, also known as 5,6-dehydro-ascomycin, disclosed as Example 8 in e.g. EP 626385 (hereinafter referred to as **Compound C**).

The 3-component solvent mixture according to the invention, as indicated above, amounts to at least 40 % w/w of the total weight of the composition, which may thus optionally contain further conventional ingredients. Alternative mixtures may amount to more than 40%, e.g. from about 55 % to almost about 100 %, from about 80 % or about 90 % to about 99.95 %, or from about 95 % to about 99.50 % of the total weight.

Components i) and ii) each independently may amount to up to about 50 %, to up to about 20 %, or to up to about 15 % of the total weight. In sum, they may amount to up to about 60 %, to up to about 40 %, or to up to about 30 % of the total weight.

Component iii) may amount to up to about 75 %, to up to about 65 % of the total weight of the composition, or from about 30 % to about 75 % of the total weight.

The active agent may be present in the compositions in an amount of from about 0.05 % to about 10 % by weight, e.g. from 0.1 % to 6 % by weight, or in the composition according to the invention from 1 % to 5 % by weight based on the total weight of the composition.

**C₃₋₈ alkanol component i)** may be a straight or branched chain saturated alkanol, e.g. isopropanol. **C₁₋₈ alkanediol component i)** may be propylene glycol (i.e. 1,2-propanediol), butylene glycol, 2-ethyl-1,3-hexanediol and hexylene glycol (i.e. 2-methyl-2,4-pentanediol), especially propylene glycol and hexylene glycol.

The ascomycin and component i) in the compositions may be present, at least initially, in a weight ratio of about 0.05 to 10 : 10 to 60, or in a weight ratio of about 0.5 to 10:50 to 60, or in a weight ratio of about 1 to 3 : 50 to 60. However, upon administration to skin the more volatile components, e.g. lower alkanols, may evaporate to yield a supersaturated solution which may contribute to further enhance penetration.

**Component ii)** may be a mono- or polyunsaturated fatty alcohol, for example a C₁₂₋₂₄, e.g. C₁₆₋₁₈ mono- or polyunsaturated fatty alcohol, an oleyl alcohol or elaidic alcohol; particularly an oleyl alcohol.

The ascomycin and component ii) in the compositions may be present in a weight ratio of about 0.05 to 10: 5 to 90, or in a weight ratio of about 0.5 to 10 : 5 to 50, or in a weight ratio of about 1 to 5: 10 to 20.

**Component iii)**a) may be a C₁₂₋₂₄, preferably C₁₄₋₁₆ carboxylic acid alkyl ester, e.g. isopropyl myristate, ethyl myristate or isopropyl palmitate, an isopropyl myristate or palmitate, or a C₂₋₁₀ alkane dicarboxylic acid alkyl ester, e.g. diisopropyl adipate or diethyl adipate, particularly diisopropyl adipate.

**Component iii)**b) may be a pharmaceutically acceptable ether diol, e.g. dipropylene glycol or diethylene glycol; a diether alcohol, e.g. diethyleneglycol mono ethyl ether; a di- or partial ether of a low molecular weight mono- or polyoxyalkanediol, e.g. tetrahydrofurfuryl alcohol polyethylene glycol ether (Glycofurol^{R}); diethylene glycol monoethyl ether (Transcutol^{R}); triethyl citrate; N-methylpyrrolidone; dimethylisosorbide (i.e. 1,4:3,6-dianhydro-2,5-di-O-methyl-D-glucitol); or propylene carbonate; particularly dimethylisosorbide.

**Component iiii)**c) may consist of medium-chained triglycerides, such as Miglyol 812^{R}.

The compositions of the invention may optionally comprise **further conventional excipients,** e.g., for easier application, a thickened solution may be desirable, e.g. a semi-solid solution or a fluid-gel or a transparent gel. This can be achieved by adding conventional **consistency agents** (viscosity-enhancing agents) to enhance the viscosity of the compositions.

Suitable **consistency agents** include e.g.:
- polyacrylic acid, as known under the names carboxypolymethylen, or carboxyvinylpolymer, or carbomer, or Carbopol^{R,}
- cellulose derivatives, including e.g. ethyl-, propyl-, methyl-, hydroxypropyl- and hydroxypropylmethyl-celluloses,
- colloidal silicon dioxide, e.g. Aerosil^{R}, e.g. Aerosil R972^{R} or Aerosil 200^{R},
- polyvinyl alcohol,
- bee wax,
- hydrogenated castor oil (Cutina HR^{R}),
- polyvinyl pyrrolidone,
- polymethylacrylate resins, e.g. Eudispert^{R} or Eudragit^{R}, and
- solid alcohols, having e.g. a C₁₂₋₂₄ chain, e.g. cetyl alcohol and/or stearyl alcohol, commercially available e.g. under the trademarks Lorol^{R} C16 and Lorol^{R} C18, respectively, from Henkel, Germany;
preferably hydroxypropylcellulose, colloidal silicon dioxide, bee wax, hydrogenated castor oil, polyvinylpyrrolidone or Eudragit^{R}.

Further conventional excipients are e.g. an **ointment base,** such as mineral hydrocarbons, e.g. liquid paraffin, petrolatum and microcrystalline wax. The ointment base, where its presence is appropriate, is in an amount of e.g. from about 0.1 % to about 40 %, e.g. from about 30 % to about 40 % of the total weight of the composition.

The compositions may also include **anti-oxidants** such as butyl-hydroxytoluene, ascorbyl palmitate, sodium pyrosulfite, butyl hydroxyanisole, propyl p-hydroxybenzoate, methyl p-hydroxybenzoate and tocopherol, as appropriate. Furthermore, the addition of preservatives such as benzyl alcohol, parabens, sorbic acid and phenyl alcohol serves to prevent bacterial growth. Where the presence of anti-oxidants and/or preservatives is appropriate, they are preferably present in an amount of from about 0.01 % to about 2.5 % w/w each.

The compositions as disclosed herein are **devoid of surfactant**, except as required during processing to e.g. a foam formulation, and are substantially free of ethanol and water, as defined above.

For the treatment of fatty or hairy skin, non-greasy formulations are preferred, such as provided in e.g. Examples 1 to 17, 22 and 23 hereunder. Absence of greasiness and low residue upon application may lead to increased convenience especially on haired skin.

The components of the compositions of the invention are described in i.a. H.P. Fiedler, "Lexikon der Hilfsstoffe für Pharmazie, Kosmetik und angrenzende Gebiete", Editio Cantor Verlag Aulendorf, Aulendorf, 4th revised and expanded edition (1996), the contents of which are hereby incorporated by reference.

The present invention relates to compositions wherein the ascomycin is e.g. pimecrolimus and
- component i) is isopropanol and/or hexylene glycol;
- component ii) is oleyl alcohol; and
- component iii) is:
   a) diisopropyl adipate and/or isopropyl myristate and/or
   b) dimethyl isosorbide and/or
   c) medium-chain triglycerides;
and optionally further conventional excipients;
especially the compositions of Examples 17 to 20 and 23, particularly Example 17 hereunder.

The compositions of the invention are indicated for use in the treatment of inflammatory and hyperproliferative skin diseases and of cutaneous manifestations of immunologically-mediated diseases. The terms "skin" and "cutaneous" should be understood broadly as comprising also diseases of e.g. nail or mucosa. Examples of immunologically-mediated diseases include alopecia areata, psoriasis, atopic dermatitis, contact dermatitis and further eczematous dermatitises, seborrhoeic dermatitis, lichen planus, pemphigus, bullous pemphigoid, epidermolysis bullosa, urticaria, angioedemas, vasculitides, erythemas, cutaneous eosinophilias, and lupus erythematous. Examples of skin diseases include dermatomyositis, leukoderma vulgaris, ichthyosis vulgaris, photoallergic sensitivity, cutaneous T cell lymphoma, acne, autoimmune diseases such as chronic rheumatoid arthritis, scleoderma and the like.

The invention further provides a composition as defined above for use in the treatment of inflammatory and hyperproliferative skin diseases and of cutaneous manifestations of immunologically-mediated diseases.

Still further, it provides the use of a composition of the invention in the preparation of a medicament for the treatment of inflammatory and hyperproliferative skin diseases and of cutaneous manifestations of immunologically-mediated diseases.

It further provides the **use of a carrier vehicle** as defined above **to enhance penetration** of pimecrolimus into human skin, nail or mucosa.

The compositions of the invention may be prepared in conventional manner by working up the components into a pharmaceutical composition. For example, the compositions of the invention may be obtained by dissolving an ascomycin in a pharmaceutically acceptable alkanol, e.g. a C₃₋₈ alkanol, a C₁₋₈ alkanediol and/or a fatty alcohol. Other components, e.g. alkane carboxylic alkyl esters and/or alkane dicarboxylic esters, and/or hydrophilic co-components, triglycerides and optional further conventional excipients, may be added at the appropriate time as is conventional.

The following Examples illustrate the invention. They are not limitative.

All percentages referred to herein are weight/weight (% w/w) except where otherwise indicated. The term "stable" should be understood to mean that no separation of components of the respective composition is observed when stored at room temperature for a period of at least 3 months or longer and that there is no decomposition of active agent. However, some unintentional crystallization may occasionally take place e.g. upon storage, and the presence of a small amount of active ingredient in crystallized form in the compositions of the invention should thus be understood to still fall within the ambit of the invention.

Chemical analysis of the active agent is undertaken using reverse phase HPLC with UV detection; λ = 210 nm. Quantification limit is 0.1 % by weight.

In Examples 1 to 25, Compound A (pimecrolimus) may be replaced with Compound B; Compound C; tacrolimus; L-733725; L-732531; and ABT-281.

### Examples 1 and 2 (solutions) and 3 (single-phase gel)

The following compositions are prepared:

| Ingredients | **Ex. 1** | **Ex. 2** | **Ex. 3** |
|---|---|---|---|
| Compound A | 1.0 | 8.0 | 1.0 |
| i) isopropanol | 49.0 | 42.0 | 47.0 |
| propylene glycol | - | - | - |
| ii) oleyl alcohol | 10.0 | 10.0 | 10.0 |
| iii) a) isopropyl myristate | 40.0 | - | 40.0 |
| iii) b) dimethylisosorbide | - | 40.0 | - |
| Further ingredients: | | | |
| hydroxypropyl cellulose (consistency agent) | - | - | 2.0 |

The compositions of Examples 1 to 3 are stable.

### Examples 4 to 9 (solutions)

The following compositions are prepared:

| Ingredients | **Ex. 4** | **Ex. 5** | **Ex. 6** | **Ex. 7** | **Ex. 8** | **Ex. 9** |
|---|---|---|---|---|---|---|
| Compound A | 1.0 | 1.0 | 1.0 | 1.0 | 1.0 | 1.0 |
| i) isopropanol | 39.0 | 39.0 | 39.0 | 39.0 | 39.0 | 39.0 |
| hexylene glycol | - | - | 10.0 | 10.0 | 10.0 | 10.0 |
| ii) oleyl alcohol | 10.0 | 10.0 | 10.0 | 10.0 | 10.0 | 10.0 |
| iii) a) isopropyl myristate | - | - | 40.0 | - | - | - |
| isopropyl palmitate | - | - | - | 40.0 | - | - |
| diisopropyl adipate | - | 50.0 | - | - | - | 40.0 |
| iii) b) dimethyl isosorbide | 50.0 | - | - | - | 40.0 | - |

The compositions of Examples 4 to 9 are stable.

### Examples 10 to 15 (solutions)

The following compositions are prepared:

| Ingredients | **Ex. 10** | **Ex. 11** | **Ex. 12** | **Ex. 13** | **Ex. 14** | **Ex. 15** |
|---|---|---|---|---|---|---|
| Compound A | 2.0 | 2.0 | 0.5 | 0.5 | 5.0 | 2.0 |
| i) propylene | 38.0 | 38.0 | - | - | - | - |
| glycol | | | | | | |
| hexylene glycol | - | - | 39.5 | 39.5 | 35.0 | 38.0 |
| ii) oleyl alcohol | 10.0 | 10.0 | 10.0 | 10.0 | 10.0 | 10.0 |
| iii) a) isopropyl | - | - | 50.0 | - | - | - |
| myristate | | | | | | |
| isopropyl | - | - | - | 50.0 | - | - |
| palmitate | | | | | | |
| diisopropyl | - | 50.0 | - | - | - | 50.0 |
| adipate | | | | | | |
| iii) b) dimethyl | 50.0 | - | - | - | 50.0 | - |
| isosorbide | | | | | | |

The compositions of Examples 10 to 15 are stable.

### Examples 16 and 17 (solutions)

The following compositions are prepared:

| Ingredients | **Ex. 16** | **Ex. 17** |
|---|---|---|
| Compound A | 1.0 | 1.0 |
| i) isopropanol | - | 10.0 |
| hexylene glycol | 5.0 | 5.0 |
| ii) oleyl alcohol | 10.0 | 10.0 |
| iii) a) diisopropyl adipate | 84.0 | 74.0 |

The compositions of Examples 16 and 17 are stable.

### Examples 18 to 21 (semi-solid ointments)

The following compositions are prepared:

| Ingredients | **Ex.18** | **Ex. 19** | **Ex. 20** | **Ex. 21 *** |
|---|---|---|---|---|
| Compound A | 1.0 | 1.0 | 1.0 | 0.3 |
| i) isopropanol | 10.0 | 10.0 | 10.0 | - |
| hexylene glycol | 5.0 | 5.0 | 5.0 | 10.0 |
| ii) oleyl alcohol | 10.0 | 10.0 | 10.0 | 10.0 |
| iii) a) diisopropyl adipate | 32.0 | 54.0 | 64.0 | - |
| iii) c) medium-chain triglycerides | - | - | - | 44.0 |
| (Miglyol 812) | | | | |
| Further ingredients: | | | | |
| liquid paraffin (ointment base) | 32.0 | - | - | 29.7 |
| colloidal silicon dioxide (Aerosil R972) | 10.0 | - | - | 6.0 |
| (consistency agent) | | | | |
| bee wax (consistency agent) | - | 20.0 | - | - |
| hydrogenated castor oil (Cutina HR) | - | - | 10.0 | - |
| (consistency agent) | | | | |
| * oleogel | | | | |

The compositions of Examples 18 to 21 are stable.

### Examples 22 to 25 (solutions)

The following compositions are prepared:

| Ingredients | **Ex. 22** | **Ex.23** | **Ex. 24** | **Ex. 25** |
|---|---|---|---|---|
| Compound A | 0.8 | 4.0 | 0.6 | 0.2 |
| i) isopropanol | 10.0 | 10.0 | 10.0 | - |
| hexylene glycol | 5.0 | 5.0 | 10.0 | 10.0 |
| ii) oleyl alcohol | 10.0 | 10.0 | 10.0 | 10.0 |
| iii) a) isopropyl myristate | - | - | - | 49.8 |
| diisopropyl adipate | - | 71.0 | - | - |
| iii) c) medium-chain triglycerides | 74.2 | - | 39.4 | - |
| (Miglyol 812) | | | | |
| Further ingredients: | | | | |
| liquid paraffin (ointment base) | - | - | 30.0 | 30.0 |

The compositions of Examples 22 to 25 are stable.

The utility of the compositions of the invention can be observed in standard clinical tests such as the test set out below using a concentration of 0.005 % to 10 % w/w (preferably 0.1 % to 3 % w/w) of the active agent.

A representative clinical trial is carried out as follows:
A randomised double-blind, vehicle-controlled within-patient study comparing the composition of the invention at a dose of 0.1 % to 3 % by weight (based on the total weight of the composition) active agent on the diseased skin area, e.g. 200 cm², corresponding to about 0.5 to 50 mg/cm², preferably 1 to 10 mg/cm² of composition, and placebo on the diseased skin area as positive control is performed in patients suffering from inflammatory and hyperproliferative skin diseases or of cutaneous manifestations of immunologically-mediated diseases.

The patients are treated with the composition twice daily for six months. The therapeutic effect is evaluated and the time to partial clearance is used for efficacy. Local tolerability of study medications and routine safety parameters, including haematology and clinical chemistry, are recorded.

The utility can also be observed using the established assay of experimentally induced allergic contact dermatitis in young domestic pigs (J. Investig. Dermatol. 98 [1992] 851-855). Several formulations were tested for inhibition of inflammatory changes (intensity and extent of redness and infiltration) by clinical examination. Efficacy was evaluated by comparison of inflammatory changes in treated and untreated contralateral test sites in the same animals. Results obtained are as follows:

| Formulation of Example No. | Efficacy vs. untreated sites (mean %; n = 12**) |
|---|---|
| 16 | 79 |
| 17 | 73 |
| 18 | 78 |
| 20 | 70 |
| Pimecrolimus cream 1 % | 68 |

| | |
|---|---|
| ** each formulation was tested in 2 test sites in 6 animals | |

The exact amount of composition of the invention to be administered depends on various factors, for example the desired duration of treatment and the rate of release of the active agent. Satisfactory results are obtained in larger mammals, for example humans, with the local application over the area to be treated of a 0.05 % to 10 % w/w, preferably 0.1 % to 3 %, concentration of the active agent once or several times a day (for example 2 to 5 times a day). In general the composition may be applied to areas of skin as small as 1 cm² to as large as 0.5 m², preferably the composition will be applied to the head area in the treatment of inflammatory and hyperproliferative skin diseases or of cutaneous manifestations of immunologically-mediated diseases. Suitable skin loadings of the active agent fall within the range of 0.005 mg/cm² to 1 mg/cm².

The compositions of the invention are found to be effective independently of the condition of the skin and are well tolerated on skin. They may be easily applied to large areas of skin using a conventional applicator, e.g. brush, cotton pad, filament, topical spray or roller ball applicator, and are thus very convenient in use.

## Claims

1. A single-phase topical liquid or semi-solid pharmaceutical composition substantially free of ethanol and water which comprises pimecrolimus in a carrier vehicle comprising a 3-component solvent mixture amounting to at least 40 % w/w of the total weight of the composition and consisting of:
- i) isopropanol and/or hexylene glycol;
- ii) oleyl alcohol; and
- iii) :
a) diisopropyl adipate and/or isopropyl myristate and/or
b) dimethyl isosorbide and/or
c) medium-chain triglycerides;
and optionally further conventional excipients

2. A composition according to claim 1 comprising further conventional excipients selected from hydroxypropyl cellulose, liquid paraffin, colloidal silicon dioxide, bee wax and hydrogenated castor oil.

3. A composition according to claim 1 wherein the pimecrolimus is present in an amount of 1 % to 5 % by weight of the composition.

4. A composition according to claim 1 which is in the form of a gel, a foam or an ointment.

5. A composition according to claim 1 for use in the treatment of inflammatory and hyperproliferative skin diseases and of cutaneous manifestations of immunologically-mediated diseases.

6. Use of a composition according to claim 1 in the preparation of a medicament for the treatment of inflammatory and hyperproliferative skin diseases and of cutaneous manifestations of immunologically-mediated diseases.

7. Use of a carrier vehicle as defined in claim 1 to enhance penetration of pimecrolimus into human skin, nail or mucosa.

## Patentansprüche

1. Einphasige topische flüssige oder halbfeste pharmazeutische Zusammensetzung, die im Wesentlichen frei von Ethanol und Wasser ist und die Pimecrolimus in einem Trägervehikel, der ein 3-Komponenten-Lösemittelgemisch, das mindestens 40 % g/g des Gesamtgewichts der Zusammensetzung ausmacht und aus
i) Isopropanol und/oder Hexylenglycol;
ii) Oleylalkohol und
iii)
a) Dilsopropyladipat und/oder Isopropylmyristat und/oder
b) Dimethylisosorbid und/oder
c) mittelkettigen Triglyceriden
besteht, umfasst,
und optional weitere herkömmliche Streckmittel umfasst.

2. Zusammensetzung nach Anspruch 1, die ferner herkömmliche Streckmittel umfasst, die aus Hydroxypropylcellulose, flüssigem Paraffin, kolloidalem Siliciumdioxid, Bienenwachs und hydriertem Rizinusöl ausgewählt sind.

3. Zusammensetzung nach Anspruch 1, wobei der Pimecrolimus in einer Menge von 1 bis 5 Gew.-% der Zusammensetzung vorhanden ist.

4. Zusammensetzung nach Anspruch 1, die in Form eines Gels, eines Schaums oder einer Salbe vorliegt.

5. Zusammensetzung nach Anspruch 1 zur Verwendung bei der Behandlung von entzündlichen und hyperproliferativen Hauterkrankungen und von kutanen Symptomen von immunologisch vermittelten Erkrankungen.

6. Verwendung einer Zusammensetzung nach Anspruch 1 bei der Herstellung eines Medikaments zur Behandlung von entzündlichen und hyperproliferativen Hauterkrankungen und von kutanen Symptomen von immunologisch vermittelten Erkrankungen.

7. Verwendung eines Trägervehikels gemäß Definition in Anspruch 1 zur Erhöhung der Penetration von Pimecrolimus in menschliche Haut, menschliche Nägel oder menschliche Mucosa.

## Revendications

1. Composition pharmaceutique liquide ou semi-solide topique à une seule phase, essentiellement dépourvue d'éthanol et d'eau, qui comprend du pimécrolimus dans un véhicule support comprenant un mélange de solvants à 3 composants représentant au moins 40% p/p du poids total de la composition et constitué de:
i) l'isopropanol et/ou l'héxylèneglycol;
ii) un alcool oléylique; et
iii)
a) l'adipate de diisopropyle et/ou le myristate d'isopropyle et/ou
b) le diméthylisosorbide et/ou
c) des triglycérides à chaînes moyennes;
et éventuellement d'autres excipients classiques.

2. Composition selon la revendication 1, comprenant des excipients classiques supplémentaires choisis parmi l'hydroxypropylcellulose, la paraffine liquide, le dioxyde de silicium colloïdal, la cire d'abeilles et l'huile de ricin hydrogénée.

3. Composition selon la revendication 1, dans laquelle le pimécrolimus est présent en une quantité de 1% à 5% en poids de la composition.

4. Composition selon la revendication 1, qui se présente sous la forme d'un gel, d'une mousse ou d'une pommade.

5. Composition selon la revendication 1 à utiliser dans le traitement des maladies inflammatoires et hyperproliférantes de la peau ou des manifestations cutanées des maladies à médiation immunologique.

6. Utilisation d'une composition selon la revendication 1 dans la préparation d'un médicament destiné au traitement des maladies inflammatoires et hyperproliférantes de la peau ou des manifestations cutanées des maladies à médiation immunologique.

7. Utilisation d'un véhicule support tel que défini dans la revendication 1 pour stimuler la pénétration du pimécrolimus dans la peau, les ongles ou la muqueuse humains.
